# EUROPEAN PATENT SPECIFICATION

(11) **EP 0 500 885 B1**
(45) Date of publication and mention of the grant of the patent: **05.03.1997**
(21) Application number: 91916235.4
(22) Date of filing: 19.08.1991
(51) Int. Cl.: C08L 3/02, C08J 5/00

(54) **SHAPED ARTICLES AS OBTAINED FROM A THERMOPLASTIC STARCH MELT**
FORMTEILE AUS EINER THERMOPLASTISCHEN STÄRKESCHMELZE
ARTICLES FORMES OBTENUS A PARTIR D'UN MELANGE EN FUSION D'AMIDON THERMOPLASTIQUE

(30) Priority: 30.08.1990 US 575464
(43) Date of publication of application: 02.09.1992
(73) Proprietor: WARNER-LAMBERT COMPANY, Morris Plains New Jersey 07950 (US)
(72) Inventor: EGLI, Markus, Dr., CH-5222 Umiken (CH); COLE, Ewart Thomas, Dr., CH-4144 Hofstetten (CH)
(74) Representative: Tesch, Rudolf, Dr.
(86) International application number: US9105873
(87) International publication number: WO9204408

(56) References cited:
- EP-A- 0 304 401
- EP-A- 0 326 517

## Description

The present invention relates to a thermoplastic melt comprising a dispersion medium consisting of a melt comprising molten starch, and a disperse phase comprising at least one hydrophilic, preferably hygroscopic, material. The terms molten starch, disperse phase, dispersion medium, hydrophilic material, and hygroscopic material are defined hereinafter.

It is known that natural starch which is found in vegetable products can be treated at elevated temperatures to form a melt. It is believed that such a melt is formed as a consequence of the starch material being heated above the glass transition and melting temperatures of its components. Preferably the starch material contains a defined amount of water and melt formation is carried out at an elevated temperature in a closed volume, and hence at an elevated pressure. It is, however, known-to form a starch melt in an open volume, essentially in the absence of water, but in the presence of a liquid having a boiling point higher than the starch glass transition and melting temperature.

Melt formation of starch is known.

European Patent Application 84300940.8 (Publication No. 118 240) and PCT Patent Application PCT/CH89/00185 (Publication No. WO 90/05161) describe methods of producing starch melts. Reference is also made to European Patent Applications 88810455.1 (Publication No. 298,920), 88810548.3 (Publication No. 304,401) and 89810046.6 (Publication No. 326,517) which further describe starch melts, methods of making such melts, and uses of same.

EP-A-0 326 517 discloses a process for forming starch into a melt, as well as shaped articles obtained therefrom. Said articles may comprise extenders, among which are to be found water-soluble polysaccharides.
EP-A-0 304 401 discloses a process of forming shaped articles from starch, wherein said articles may further comprise extenders, among which are to be found water-soluble polysaccharides.
Both documents are directed to the use of water-soluble material.

Starch contained in starch melts and in articles made therefrom is often called "destructurised" starch or "destructured" starch.

Different degrees of destructurisation are possible which can be measured by various methods. One method, for example, is to measure the remaining amount of granular structure which is contained in the destructurised starch. This can be determined by known microscopic methods. It is an advantage of the present invention that articles possessing excellent physical properties may be formed from starch which has a relatively low degree of destructurisation, i.e wherein uniform thermoplastic melt formation per se is sufficient.

It is preferred that the destructured starch used in the present invention has been heated to a high enough temperature and for a time sufficient so that specific endothermic transition analysis as represented by differential scanning calorimetry indicates that a specific relatively narrow peak just prior to oxidative and thermal degradation has disappeared, as is described in European Patent Application No. 89810046.6 (Publication No. 326 517)

The term "destructurised" starch will be used herebelow, and is thus to be construed accordingly.

An important property of destructurised starch is its capability to take up water and to disintegrate as a consequence thereof. For many purposes the rate of disintegration of articles made from such destructurised starch is insufficient. Thus, for example, pharmaceutical containers which are fabricated from such destructurised starch in general exhibit a lower dissolution rate than pharmaceutical containers in the form of dip moulded hard gelatin capsules.

The present invention provides a solution to the aforementioned insufficiency.

According to the present invention, there is provided shaped articles as defined in claim 1.

The invention particularly provides shaped articles in the form of a member selected from the group consisting of bottles, sheets, films, packaging materials, pipes, rods, laminated films, sacks, bags, foams, granules and powders.

The invention most particularly provides shaped articles in the form of pharmaceutical containers, especially adapted for use as a carrier material for pharmaceutically and veterinarilly active compounds, as well as for numerous non-pharmaceuticals which are filled in such pharmaceutical containers.

The invention still further provides a process as defined in claim 13.

The terms "dispersion medium" and "disperse phase" within the context of the invention are to be construed in accord with the following: The thermoplastic melt of the present invention consists of material comprised by a medium and material comprised by a phase. It is a requirement of the present invention that the phase be incompletely miscible with the medium when both are present in the thermoplastic melt i.e. that the phase is dispersed in, but not dissolved in, the material comprised by the dispersion medium. The phase may thus include course particles having a particle size of from about 100 to about 500um, or particles of smaller size (from about 10 to about 100um) to form colloidal systems, or larger particles when such behave likewise when present in the thermoplastic melt.

The term polymer b) within the context of this invention means a material which is water-swellable, i.e. a material which is able to take up water at room temperature in an amount of at least 10 grams per 100 grams of material, and preferably in an amount of at least 20 grams per 100 grams of material, which readily absorbs and/or adsorbs and retains moisture from the environment at room temperature, but does not liquify due to dissolution of said material by absorption, adsorption and retention of said moisture. Such material may absorb and/or adsorb and retain water in an amount of up to 1000 times its own weight.

The term component b) as used herein includes hygroscopic materials.

The disperse phase is preferably comprised by a fibrous polymer selected from the group consisting of polyvinylpyrrolidone and polysaccharides such as cellulose, chitin, starch or chitosan, which preferably are cross-linked and in which some of the hydroxyl groups of the anhydroglucose groups are substituted.

The average particle size of the fibrous polymers of the disperse phase is preferably in the range of from about 10 to about 500um. More preferred such average particle sizes are from about 20 to about 300um, and most preferred such sizes are from about 20 to about 100um.

Such polymers have been previously used at concentrations of about 2% to about 6% as a disintegrant in tablets made by cold compression of dry components. When used in wet processing, such as the dip moulding of hard gelatin capsules, such materials do not promote disintegration of the capsule indicating that when processed in the presence of moisture such polymers substantially lose their capacity to act as disintegrants.

When water is added to starch mixed with such polysaccharide fibres, it is assumed that such fibres absorb enough water to render them ineffective as disintegrant materials. In addition, said disperse phase material is present in a relatively low concentration and under conditions of melt formation is enveloped entirely by the starch medium. The disperse phase material remains in this enveloped form once the melt has cooled and is solidified. It is therefore surprising to find that solidified thermoplastic starch melts incorporating such materials exhibit excellent dissolution characteristics.

The component b), which may be present in the thermoplastic melt at a concentration of up to about 4%, preferably up to about 2%, by weight based on the weight of the thermoplastic melt, may comprise a polymer of cellulose, chitin, chitosan and starch, which may be substituted and/or cross linked as mentioned above.

Said component b) may also be a polyvinylpyrrolidone polymer.

The thermoplastic melt may further comprise at least one member of the group consisting of fillers, lubricants, mould release agents, plasticisers, stabilisers and colouring agents.

The invention is further apparent from the following description.

The starch which is used to form the melt of destructurised starch includes at least one member of the group consisting of chemically non-modified starches of vegetable origin, which starches are composed mainly of amylose and/or amylopectin and are derived from potatoes, rice, tapioca, corn, pea, rye, oats and wheat. Such starch includes pre-extruded starches; physically modified starch; irradiated starch; and starch in which divalent ions associated with phosphate groups therein have been removed, either partly or wholly, and optionally replaced, either partly or wholly, by different divalent ions or with mono or polyvalent ions.

It has been found that starches with a water content in the range of about 5 to 40% by weight, based on the weight of the starch water composition, when heated at elevated temperatures and in a closed vessel exhibit a specific narrow endothermic transition prior to the endothermic transition characterising its oxidative and thermal degradation. Such a specific narrow endothermic transition may be determined by differential scanning calorimetric analysis (DSC) and is indicated on a DSC diagram by a specific relatively narrow peak substantially immediately prior to the endotherm characteristic of its oxidative and thermal degradation. This peak disappears as soon as the specific endothermic transition has been traversed. The term "starch" as used herein thus includes starches, so treated as to have traversed this specific endothermic transition.

The term "starch" further includes starch derivatives, i.e. substituted starches with a degree of substitution of up to about 1. Such substituents are preferably hydroxyalkyl such as hydroxyethyl or hydroxypropyl, and said starches are preferably thus substituted to a degree of 0.05 to 0.5, and more preferably from 0.05 to 0.25. In this sense, the starches described above may be either wholly or partly replaced with such starch derivatives.

Preferably the starch is destructurised in the presence of water which may be present in the thermoplastic melt at between about 5 and 40%, and preferably at between about 5 to 30% by weight, based on the weight of the thermoplastic melt.

In order to work with the material near to the equilibrium water content which exists when such is exposed to a free atmosphere, however, the starch material has a water content of between about 10 and 20% by weight, and preferably of between about 14 and 18% by weight, based on the weight of the thermoplastic melt.

The starch component of the thermoplastic melt includes destructurised starch prepared by other methods, for example, in the absence of water.

The polymer b) of the disperse phase preferably has a melting point which is higher than the glass transition temperature of the destructurised starch and preferably absorbs up to a thousand times its own weight in water. The precise amounts of water absorbed vary according to the nature of the material and the ionic strength of the aqueous environment. For example, the amount of water absorbed by the polymer b) is generally reduced as the ionic strength in which it is situated is increased.

The material comprises a polymer of glucose moieties, and preferably is selected from the group consisting of cellulose, chitin, starch and chitosan.

Preferably, at least some of the hydroxyl groups of anhydroglucose moieties comprised by the polymer are substituted, with, for example, a member from the group consisting of carboxy-carboxyalkyl, sulfoalkyl, and salts thereof, and dialklyaminoalkyl, - or quaternary derivative thereof. Such derivatives are, for example, carboxymethylcellulose, diethylaminoethyl cellulose, triethanolamine cellulose, polyethyleneimine cellulose, and carboxymethyl starch.

In the preferred embodiment, the disperse phase is comprised by the compounds mentioned above, which are internally cross linked. These polymers are preferably substituted to a degree of between 0.5 and 1.2 and preferably are substituted to a degree of about 0.7. Preferably such materials are of a strand or fibrous type, and have an average strand or average fibre length of from 10 to 500µm. More preferred such average strand or fibre sizes are from 20 to 300µm, and most preferred such sizes are from 20 to 100µm. Said materials are preferably cross linked to such an extent that they are substantially water-insoluble. Preferred cross linked materials include cross-linked carboxymethyl starch, internally cross-linked carboxymethyl cellulose and salts thereof, and cross-linked cellulose amines possessing tertiary or quaternary amino groups.

Internally cross linked sodium carboxymethyl cellulose is listed in the US-National Formulary as Croscarmellose Sodium Type A as sold by FMC Corporation (Philidelphia, USA) under the Trade name Ac-Di-Sol. The average strand or fibre size of said cross-linked carboxymethyl cellulose is from about 70 to about 80µm, and it has been used previously at concentrations of between 2 and 6% as a disintegrant in tablets and capsules made by a cold compression process. A molten system comprising destructurised starch, where generally temperatures of between 160 and 180°C as well as high pressures are used is considerably different from a conventional pharmaceutical tablet manufactured at room temperature.

Chemically modified suitable starches comprised by the material, such as carboxymethyl starch or sodium starch glycolate are available under the Trade names of Explotab (Edward Mendell Company Incorporated Carmel, New York) and Primojel (Generichem Corp. Little Falls, New Jersey). The average fibre or strand size of said modified starches is about 70µm.

A further material suitable as the component b) of the disperse phase is microcrystalline cellulose as sold under the trade name Avicel PH-101 by Fluka Chemie AG of Industriestrasse 25, CH-9470 Buchs, Switzerland. The average fibre or strand size of said microcrystalline cellulose is from about 20 to about 100µm, depending upon the grade used.

A still further material suitable as the component b) of the disperse phase is a cross linked polyvinylpyrrolidone as sold under the Trade name Polyplasdone XL (GAF Corp., New York), or Kollidon. The average fibre or strand size of said polyvinylpyrrolidone depends on the precise grade used, but is typically in the range of from about 20 to about 250µm.

Pullulan, or pullulan derivatives, which are, or are rendered substantially insoluble in starch, by cross linking or acetylation, for example, may be used as the material of the disperse phase.

The pullulan or derivative may be substituted to between about 0.5 and 1.0.

The component b) is present in the thermoplastic melt at a concentration of between about 0.1 and 4%, by weight, based on the weight of the thermoplastic melt. Preferably the material is present in the thermoplastic melt at a lower concentration of between about 0.3 and about 2%, by weight, based on the weight of the thermoplastic melt, and most preferably is present in the thermoplastic melt at a concentration of between about 0.5 and about 1%, by weight, based on the weight of the thermoplastic melt.

Starch may be mixed with the hydrophilic material and optionally other additives as mentioned hereinbelow in any desired sequence. For example, the starch may be mixed with all of the intended additives to form a blend, which blend may then be heated to form a thermoplastic melt.

The starch may, however, be mixed with optional additives, the starch destructurised and granulated before addition of the hydrophilic material, which mix may then be further processed.

Preferably, however, the starch is mixed with additives together with the component b) to form a free flowing powder, which is useful for continuous processing, and destructurised and either granulated or moulded directly into a shaped article, for example a pharmaceutical container.

Thus, the starch and all of the intended additives can be mixed in a conventional mixer, the water content of the starch being adjusted to between 5 and 40%. The thus formed mixture is passed through an extruder to produce granulates or pellets as one form of shaped article suitable for further processing. It is, however, possible to avoid the granulation step and to process the obtained melt directly using down stream equipment to produce pharmaceutical capsules, other containers, films (including blown films), sheets, profiles, pipes, tubes and other shaped articles. The sheets may be used subsequently in thermoforming processes.

In order to destructurise the starch, and / or form a thermoplastic melt according to the invention, it is suitably heated in a screw and barrel of an extruder for a time sufficient to enable uniform melt formation of the starch so that the starch is destructurised. The temperature is preferably within the range of 105°C to 240°C, and more preferably within the range of from 130 to 190°C, the precise temperature being dependent up on the type and nature of the starch used. It is preferred that potato or corn starch is used. For thermoplastic melt formation according to the invention, the composition preferably is heated in a closed volume, such as a closed vessel, or in the finite volume created by the sealing action of unmolten feed material, which action is apparent in the screw and barrel of injection moulding equipment.

Thus the screw and barrel of an injection moulding machine, or an extruder is to be understood as being a closed vessel. Pressures created in such an enclosed volume correspond to the vapour pressure of water at the used temperature. It will be appreciated that pressures may be applied or generated as is known to be possible in the use of said screw and barrel.

The preferred applied and/or generated pressures are in the range of pressures which occur in extrusion and are known per se, being up to 150 x 10⁵N/m², preferably up to 75 x 10⁵N/m² and most preferably up to 50 x 10⁵N/m². The thus obtained destructurised starch composition is granulated and is ready to be mixed with further components according to a chosen mixing and processing procedure to obtain a granular mixture of destructurised starch starting material to be fed to the screw barrel.

The melt obtained in the screw and barrel may, however, be injection moulded directly into a suitable mould, i.e directly further processed to a final product if all desired components are already present.

Within the screw the granular mixture is heated to a temperature which is generally within the range of about 80 to about 200°C, preferably within the range of about 120 to about 190°C and most preferably within the range of about 130 to about 190°C.

The minimum pressures under which the melts are formed correspond to the water vapour pressures produced at said temperatures. The process is carried out in a closed vessel, as defined above, and a range of pressures suitable for extrusion or injection moulding processes are from zero to 150 x 10⁵N/m², preferably from zero to 75 x 10⁵N/m² and most preferably from zero to 50 x 10⁵N/m².

In forming an injection moulded article with the thermoplastic melt of the present invention, the range of pressures customarily used in injection moulding are used, viz, from about 300 x 10⁵N/m² to about 3000 x 10⁵N/m². It is preferred, however, that pressures from about 700 x 10⁵N/m² to about 2000 x 10⁵N/m² are used in the injection moulding process.

Preferably the thermoplastic melt comprises at least one member selected from the group consisting of fillers, lubricants, mould release agents, plasticisers, stabilisers and colouring agents.

Suitable fillers include, for example, oxides of magnesium, aluminium, silicon, and titanium. The fillers are present in the thermoplastic melt at a concentration of up to about 20% by weight, and preferably between about 3.0 and about 10%, by weight, based on the weight of the thermoplastic melt.

The lubricants include stearates of aluminium, calcium, magnesium, and tin, as well as magnesium silicate, silicones and substances which function in like manner. The lubricants are present in the thermoplastic melt in concentrations of about 0.1 to about 5% by weight, and preferably at about 0.1 to about 3% by weight, based on the weight of the melt.

Plasticisers include low molecular weight poly(alkylene oxides), such as, for example, poly(ethylene glycols), poly(propylene glycols) poly(ethylene-propylene glycols), organic plasticisers of low molecular mass, such as, for example, glycerol; pentaerythritol; glycerol monoacetate, diacetate, or triacetate; propylene glycol; sorbitol; sodium diethylsulfosuccinate; triethyl citrate and tributyl citrate and other substances which function in like manner. Such plasticisers are preferably present in the thermoplastic melt at a concentration of between about 0.5% and about 25% by weight, and more preferably between about 0.5% and about 10% by weight, based on the weight of all of the components, including the water therein.

Preferably the sum of the plasticiser and water content of the thermoplastic melt does not exceed about 25% by weight, and most preferably does not exceed about 20% by weight, based on the weight of the thermoplastic melt.

Stabilisers include antioxidants such as thiobisphenols, alkylidenbisphenols, secondary aromatic amines; stabilisers against photo-decomposition, such as, for example, uv absorbers and quenchers; hydroperoxide decomposers; free radical scavengers, and anti-microbial agents.

Colouring agents include known azo dyes, organic or inorganic pigments, or colouring agents of natural origin. Inorganic pigments are preferred, such as the oxides of iron or titanium, these oxides being present in the thermoplastic melt at a concentration of between about 0.1 and about 10% by weight, and preferably present at a concentration of between about 0.5 and about 3% by weight, based on the weight of the thermoplastic melt.

Still further substances which may be added to the starch material of the thermoplastic melt include animal or vegetable fats, preferably in their hydrogenated forms, especially those which are solid at room temperature. Such fats preferably have a melting point of at least 50°C and include triglycerides of C12-, C14-, C16-, and C18- fatty acids.

The fats are added to the material comprising the thermoplastic melt alone without extenders or plasticisers, or to the melt together with mono- or di- glycerides or phosphatides, of which lecithin is preferred. Said mono- and diglycerides are preferably derived from said animal or vegetable fats.

The total concentration of said fats, mono-, di- glycerides and phosphatides may be up to 5% by weight, based on the weight of the thermoplastic melt.

The materials described herein above form thermoplastic melts on heating under conditions of controlled water content and temperature and pressure. Such melts may be processed in the manner used for conventional thermoplastic materials, such as injection moulding, blow moulding, extrusion, coextrusion, compression moulding, vacuum forming, and thermoforming to produce shaped articles.

Such articles include containers, bottles, sheets, films, packaging materials, pipes, rods, laminated films, sacks, bags, foams, granules and powders.

Particularly preferred articles are pharmaceutical containers, in the form of capsules, especially when such are used as a carrier material for pharmaceutically and veterinarilly active compounds.

Such compounds include biocides, such as insecticides and pesticides, where it is desired subsequently to release rapidly such compounds therefrom.

The invention will be further apparent from a consideration of the following examples.

### Example 1

Drug containers were prepared both with and without a disperse phase.
(a) Drug Container Composition without Disperse phase
   81.0 parts of natural potato starch, 1 part of a lubricant/mould release agent (hydrogenated fat), 0.5 parts of a melt flow accelerator (lecithin), 0.5 parts of an opacifier (titanium dioxide) and 17 parts of water were mixed in a powder mixer for 10 minutes to form a free flowing powder. The hydrogenated triglyceride contained the fatty acids C18:C16:C14 in the ratio of 65:31:4 respectively.
   The thus formed powder was fed into a hopper and thence to a screw barrel having a temperature of 170 to 180°C and an applied pressure of 55 to 75 bar where it was maintained for 7 minutes. It was then injected at an injection moulding pressure of 1400 bar into an injection moulding tool for the production of capsule body and cap parts, the mould wall of which tool was maintained at 40°C. After cooling and ejection from the mould, dimensionally stable capsule body and cap parts were obtained, which could easily be processed on a filling machine.
(b) Drug Container Composition containing Disperse Phase
   80.25 parts of natural potato starch, 1 part of hydrogenated fat, 0.5 parts of lecithin, 0.5 parts of titanium dioxide, 0.75 parts of disperse phase in the form of a dry powder, and 17 parts of water were mixed in a powder mixer for 10 minutes to form a free flowing powder. The hydrogenated triglyceride contained the fatty acids C18:C16:C14 in the ratio of 65:31:4 respectively. The disperse phases, which were present at 0.75% by weight, based on the weight of the thermoplastic melt, were (1) Croscarmellose sodium; (2) cross linked polyvinylpyrrolidone; (3) carboxymethyl cellulose; (4) carboxymethyl starch; and (5) microcrystalline cellulose (Avicel). The thus formed powder was fed into a hopper and thence to a screw barrel at an applied pressure of 55 to 75 bar where it was maintained for 7 minutes. For (1) the temperature of the barrel was 170 to 180°C; for (2) the temperature was 160 to 170°C; for (3) the temperature was 155 to 165°C; for (4) the temperature was 160 to 170°C and for (5) the temperature was 150 to 160°C. The thus formed melts were then injected at an injection moulding pressure of 1400 bar for (2) and (4); 1200 bar for (1); 1600 bar for (3); and 1500 bar for (5), into an injection moulding tool for the production of capsule body and cap parts, the mould wall of which was maintained at 40°C. After cooling and ejection from the mould, dimensionally stable capsule body and cap parts were obtained, which could easily be processed on a filling machine.

The containers from (a) and (b) above were filled with identical formulations of paracetamol powder comprising 85.54 parts paracetamol, 2.5 parts of maize starch, 11.7 parts of microcrystalline cellulose (Avicel) and 0.26 parts of hydrogenated cottonseed oil. The containers were filled with the paracetamol formulation using a Bosch (CAPILL) filling machine, which machine additionally sealed the body and cap parts of the capsule.

The dissolution rates of paracetamol from the two samples produced as described under (a) and (b) above were determined using the standard USP test procedure for paracetamol capsules. The results of the determination are given in Table 1.

The pharmaceutical container in Table 1 is given as CAPILL (Trade name).

**Table 1**

| | % Paracetamol dissolved after | | |
|---|---|---|---|
| | 15 min | 30 min | 45 min |
| CAPILL without | | | |
| Disperse Phase | 30 | 56 | 82 |
| CAPILL with 0.75 % | | | |
| Croscarmellose sodium | | | |
| as disperse phase | 84 | 96 | 100 |
| CAPILL with 0.75 % | | | |
| cross linked polyvinyl | | | |
| polypyrrolidone as | | | |
| disperse phase | 71 | 89 | 94 |
| CAPILL with 0.75 % | | | |
| carboxy methyl cellulose | | | |
| (not cross-linked) | | | |
| as disperse phase | 83 | 95 | 99 |
| CAPILL with 0.75 % | | | |
| carboxy methyl starch | | | |
| as disperse phase | 97 | 100 | |
| CAPILL with 0.75 % | | | |
| microcrystalline cellulose | | | |
| (Avicel) as disperse phase | 85 | 97 | 100 |

### Example 2

Example 1 was repeated with the aim of comparing a capsule made without a disperse phase and with the addition of croscarmellose sodium as the disperse phase to the thermoplastic melt from which the capsule walls are made.

The 85.5 parts of paracetamol of Example 1 were replaced by 85.5 parts of aspirin and the standard USP test procedure for aspirin capsules was used. The results are given in Table 2.

**Table 2**

| | % Aspirin dissolved after | | |
|---|---|---|---|
| | 15 min | 30 min | 45 min |
| CAPILL without | | | |
| Croscarmellose sodium | 4 | 9 | 17 |
| CAPILL with 0.75 % | | | |
| Croscarmellose sodium | 57 | 74 | 85 |

### Example 3

In a further test, the aspirin dissolution rate was measured using a modified USP test procedure in which 0.5µg of alpha amylase was added to the dissolution medium and the basket stirring rate was increased from 100 to 150 rpm. The results obtained are given in Table 3.

**Table 3**

| | % Aspirin dissolved after | | |
|---|---|---|---|
| | 15 min | 30 min | 45 min |
| CAPILL without | | | |
| Croscarmellose sodium | 19 | 44 | 57 |
| CAPILL with 0.7% | | | |
| Croscarmellose sodium | 70 | 84 | 91 |
| CAPILL with 1.0% | | | |
| Croscarmellose sodium | 68 | 84 | 91 |
| CAPILL with 1.5% | | | |
| Croscarmellose sodium | 69 | 85 | 91 |

### Example 4

Example 3 was repeated, but the disperse phase material was cross linked polyvinylpolypyrrolidone, present in a concentration of 3% by weight of the empty pharmaceutical capsule. The results are given in Table 4.

**Table 4**

| | % Acetaminophen dissolved after | | |
|---|---|---|---|
| | 15 min | 30 min | 45 min |
| CAPILL without | | | |
| Croscarmellose sodium | 38 | 82 | 93 |
| CAPILL with 3.0% | | | |
| cross linked polyvinyl | | | |
| polypyrrolidone | 70 | 95 | 100 |

The addition of component b), as disclosed herein, to the starch composition forming the destructurised starch melt in quantities similar to those found in tablets, i.e at concentrations of from 2 to 6% yields a material which behaves relatively poorly in melt formation and injection moulding processes. The addition of relatively minor quantities, 0.75% for example, of such materials to the starch composition forming the destructurised starch melt has the surprising effect of being useful in the melt formation process as well as in increasing considerably the dissolution rate of a drug added to a container produced therefrom.

## Claims

1. A shaped article obtained from a thermoplastic melt, wherein said thermoplastic melt comprises:
a) molten destructurized starch or starches substituted with hydroxyalkyl up to a degree of substitution of up to 1 or mixtures thereof, wherein the starch or substituted starches are molten at a temperature of 105°C to 240°C under a pressure of up to 150 x 10⁵ N/m², having a water content from 5 to 40% by weight related to the melt, and
b) dispersed therein 0.1 to 4% by weight of at least one polymer with a melting point which is higher than the glass transition temperature of the destructurised starch, said polymer being able to take up water at room temperature in an amount of at least 10 grams per 100 grams of said polymer without liquefying due to dissolution

2. A shaped article according to claim 1, wherein component b) is selected from polysaccharides or cross-linked polyvinylpyrrolidone.

3. A shaped article according to claim 1 or 2 wherein component a) is selected from starch of vegetable origin, pre-extruded starch, irradiated starch or starch substituted by hydroxyalkyl groups to a degree of 0.05 to 0.5, and component b) is selected from cellulose, chitin, starch, chitosan, all unsubstituted or substituted by carboxy, carboxyalkyl, sulfoalkyl, dialkylaminoalkyl and salts thereof.

4. A shaped article according to claims 1, 2 or 3 wherein component b) is selected from carboxymethyl cellulose and salts thereof, diethylaminoethyl cellulose and salts thereof, triethanolamine cellulose and salts thereof, polyethyleneimine cellulose and salts thereof, carboxymethyl starch and salts thereof, cross-linked carboxymethyl cellulose and salts thereof, internally cross-linked carboxymethyl cellulose and salts thereof or cross-linked cellulose amines possessing tertiary or quarternary amino groups.

5. A shaped article according to claims 1 to 4, wherein component a) is derived from potato starch and component b) crosslinked polyvinylpyrrolidone, carboxymethyl starch, internally cross linked carboxymethyl cellulose or microcrystalline cellulose.

6. A shaped article according to claims 1 to 5, wherein component a) contains 5 to 30% by weight water, preferably 10 to 20%, most preferably 14 to 18%, and component b) is contained in an amount of 0.3 to 2% by weight, preferably 0.5 to 1%.

7. A shaped article according to claims 1 to 6, wherein component b) is a fibrous polymer with an average fiber length of 20 to 300 µm, preferably 20 to 100 µm.

8. A shaped article according to claims 1 to 7, wherein component b) is 0.5 to 1% croscarmellose sodium.

9. A shaped article according to claims 1 to 8, wherein the melt contains further components selected from fillers, lubricants, mould release agents, plasticisers, stabilisers and colouring agents.

10. A shaped article according to claims 1 to 9 comprising containers, bottles, sheets, films, packaging materials, pipes, rods, laminated films, sacks, bags, foams, granules and powders.

11. A shaped article according to claim 10 which is a pharmaceutical capsule.

12. A shaped article according to claim 10 or 11 used as a carrier material for pharmaceutical or veterinarily active compounds.

13. A process for producing shaped articles according to claims 1 to 12 comprising
a) melting component a) of claim 1 to 12 at a temperature of 105°C to 240°C under a pressure of up to 150 x 10⁵ N/m²,
b) admixing component b) of claim 1 to 12 before, during or after the melting process to obtain a dispersion, and
c) forming the dispersion by injection moulding, blow moulding, extrusion, coextrusion, compression moulding, vacuum forming, foaming or thermofoaming to the desired products.

## Patentansprüche

1. Formkörper, erhalten aus einer thermoplastischen Schmelze, welche
a) aufgeschmolzene entstrukturierte Stärke und/oder bis zu einem Substitutionsgrad von bis zu 1 hydroxyalkyl-substituierte Stärkederivate, die bei einer Temperatur von 105°C bis 240°C unter einem Druck von bis zu 150 x 10⁵ N/m² aufgeschmolzen wurde(n) und einen Wassergehalt in bezug auf die Schmelze von 5 bis 40 Gew.-% aufweist (aufweisen), und
b) 0,1 bis 4 Gew.-% an mindestens einem (darin) dispergierten Polymer mit einem Schmelzpunkt oberhalb der Einfriertemperatur der entstrukturierten Stärke, das bei Raumtemperatur ohne Verflüssigung infolge Auflösens Wasser in einer Menge von mindestens 10 Gramm pro 100 Gramm Polymer aufzunehmen vermag,
umfaßt.

2. Formkörper nach Anspruch 1, wobei die Komponente b) aus Polysacchariden oder vernetztem Polyvinylpyrrolidon ausgewählt ist.

3. Formkörper nach Anspruch 1 oder 2, wobei die Komponente a) aus Stärke pflanzlichen Ursprungs, vorextrudierter Stärke, bestrahlter Stärke oder mit Hydroxyalkylgruppen bis zu einem (Substitutions-)Grad von 0,05 bis 0,5 substituierter Stärke und die Komponente b) aus Cellulose, Chitin, Stärke, Chitosan, sämtliche unsubstituiert oder durch Carboxy, Carboxyalkyl, Sulfoalkyl oder Dialkylaminoalkyl substituiert, und deren Salzen ausgewählt sind.

4. Formkörper nach Anspruch 1, 2 oder 3, wobei die Komponente b) aus Carboxymethylcellulose und deren Salzen, Diethylaminoethylcellulose und deren Salzen, Triethanolamincellulose und deren Salzen, Polyethylenimincellulose und deren Salzen, Carboxymethylstärke und deren Salzen, vernetzter Carboxymethylcellulose und deren Salzen, intern vernetzter Carboxymethylcellulose und deren Salzen oder vernetzten Celluloseaminen mit tertiären oder quaternären Aminogruppen ausgewählt ist.

5. Formkörper nach Ansprüchen 1 bis 4, wobei die Komponente a) aus Kartoffelstärke und die Komponente b) aus vernetztem Polyvinylpyrrolidon, Carboxymethylstärke, intern vernetzter Carboxymethylcellulose oder mikrokristalliner Cellulose herrühren.

6. Formkörper nach Ansprüchen 1 bis 5, wobei die Komponente a) 5 bis 30, zweckmäßigerweise 10 bis 20, vorzugsweise 14 bis 18 Gew.-% Wasser enthält und die Komponente b) in einer Menge von 0,3 bis 2, vorzugsweise 0,5 bis 1 Gew.-%, vorhanden ist.

7. Formkörper nach Ansprüchen 1 bis 6, wobei die Komponente b) aus einem faserförmigen Polymer mit einer durchschnittlichen Faserlänge von 20 bis 300, vorzugsweise 20 bis 100 µm, besteht.

8. Formkörper nach Ansprüchen 1 bis 7, wobei die Komponente b) aus 0,5 bis 1% Croscarmellose-Natrium besteht.

9. Formkörper nach Ansprüchen 1 bis 8, wobei die Schmelze weitere Komponenten, ausgewählt aus Füllstoffen, Gleitmitteln, Formtrennmitteln, Plastifizierungsmitteln, Stabilisatoren und Färbemitteln, enthält.

10. Formkörper nach Ansprüchen 1 bis 9, umfassend Behälter, Flaschen, Folien, Filme, Verpackungsmaterialien, Rohre, Stäbe, Verbundfilme, Säcke, Beutel, Schaumstoffe, Granulate und Pulver.

11. Formkörper nach Anspruch 10 in Form einer Arzneimittelkapsel.

12. Formkörper nach Anspruch 10 oder 11 zur Verwendung als Trägermaterial für pharmazeutische oder veterinärmedizinisch aktive Verbindungen.

13. Verfahren zur Herstellung von Formkörpern nach Ansprüchen 1 bis 12 durch
a) Aufschmelzen der Komponente a) nach Ansprüchen 1 bis 12 bei einer Temperatur von 105°C bis 240°C unter einem Druck von bis zu 150 x 10⁵ N/m²,
b) Zumischen der Komponente b) nach Ansprüchen 1 bis 12 vor, während oder nach dem (des) Aufschmelzen(s) zur Bildung einer Dispersion und
c) Ausformen der Dispersion durch Spritzguß, Blasformen, Extrudieren, Koextrudieren, Formpressen, Vakuumformen, Aufschäumen oder Thermoschäumen zu den gewünschten Produkten.

## Revendications

1. Un article façonné obtenu à partir d'une masse fondue thermoplastique, ladite masse fondue thermoplastique comprenant:
(a) de l'amidon déstructuré fondu ou des amidons substitués par des hydroxyalkyl jusqu'à un degré de substitution pouvant atteindre un (1) ou leurs mélanges, dans lesquels l'amidon ou les amidons substitués sont fondus à une température comprise entre 105 et 240° C sous une pression pouvant atteindre 150 x 10⁵ N/m², présentant une teneur en eau comprise entre 5 et 40% en poids par rapport au poids de la masse fondue, et
(b) la dispersion dans la masse obtenue de 0,1 à 4% en poids d'au moins un polymère présentant un point de fusion qui est supérieur à la température de transition vitreuse de l'amidon déstructuré, ce polymère étant susceptible d'absorber de l'eau à température ambiante en une quantité d'au moins 10 grammes pour 100 grammes dudit polymère, sans que ne se produite une liquéfaction due à la dissolution.

2. Un article façonné selon la revendication 1, dans lequel le constituant b) est choisi parmi les polysaccharides ou les polyvinyl-pyrrolidones réticulés.

3. Un article façonné selon la revendication 1 ou 2, dans lequel le constituant a) est choisi dans le groupe comprenant l'amidon d'origine végétale, l'amidon préextrudé, l'amidon irradié ou l'amidon substitué par les groupements hydroxyalkyl jusqu'à un degré de 0,05 à 0,5, et le constituant b) est choisi dans le groupe comprenant cellulose, chitine, amidon, chitosan, qu'ils soient non substitués ou substitués par des radicaux carboxy, carboxyalkyl, sulfoalkyl, dialkylaminoalkyl, ainsi que les sels en dérivant.

4. Un article façonné selon la revendication 1, 2 ou 3, dans lequel le constituant b) est choisi dans le groupe comprenant la carboxyméthyl cellulose et les sels en dérivant, la diéthylaminoéthyl cellulose et les sels en dérivant, la triéthylaminoéthyl cellulose et les sels en dérivant, le polyéthylèneimine cellulose et les sels en dérivant, le carboxyméthyl amidon et les sels en dérivant, la carboxyméthyl cellulose réticulée et les sels en dérivant, la carboxyméthyl cellulose réticulée interne et les sels en dérivant, ou les cellulose amines réticulées présentant des groupes amino tertiaires ou quaternaire.

5. Un article façonné selon l'une quelconque des revendications 1 à 4, dans lequel le constituant a) provient d'amidon de pomme de terre et le constituant b) consiste en polyvinyl pyrrolidone réticulé, carboxyméthyl amidon, carboxyméthyl cellulose réticulée interne ou cellulose microcristalline.

6. Un article façonné selon l'une quelconque des revendications 1 à 5, dans lequel le constituant a) contient 5 à 30% en poids d'eau, de préférence 10 à 20% et plus particulièrement 14 à 18% et le constituant b) est présent en une quantité de 0,3 à 2% en poids, de préférence de 0,5 à 1%.

7. Un article façonné selon l'une quelconque des revendications 1 à 6, dans lequel le constituant b) est un polymère fibreux présentant une longueur de fibre moyenne de 20 à 300 µm, de préférence de 20 à 100 µm.

8. Un article façonné selon l'une quelconque des revendications 1 à 7, dans lequel le constituant b) est formé de 0,5 à 1% de croscarmellose sodium.

9. Un article façonné selon l'une quelconque des revendications 1 à 8, dans lequel la masse fondue contient en outre des constituants choisis dans le groupe comprenant des charges, des lubrifiants, des agents de démoulage, des plastifiants, des stabilisants et des colorants.

10. Un article façonné selon l'une quelconque des revendications 1 à 9, consistant en récipients, bouteilles, feuilles, films, matériaux d'emballage, conduites, tiges, films stratifiés, sacs, pochettes, mousses, granules et poudres.

11. Un article façonné selon la revendication 10, consistant en une capsule pharmaceutique.

12. Un arcticle façonné selon la revendication 10 ou 11, utilisé en tant que matériau support de composé actif du point de vue pharmaceutique ou vétérinaire.

13. Un procédé de production d'articles façonnés selon l'une quelconque des revendications 1 à 12, comprenant:
a) la fusion du composant a) selon l'une des revendications 1 à 12, à une température comprise entre 105° et 240°C sous une pression pouvant atteindre 150 x 10⁵ N/m²,
b) l'addition du composé b) selon l'une des revendications 1 à 12 précitées, avant, durant ou après l'opération de fusion, pour conduire à l'obtention d'une dispersion, et
c) le formage de la dispersion par moulage par injection, moulage par soufflage, extrusion, coextrusion, moulage par compression, formage sous vide, moussage ou thermomoussage, en vue de l'obtention du produit recherché.
